# EUROPEAN PATENT APPLICATION

(11) **EP 2 314 283 A1**
(43) Date of publication of application: **27.04.2011**
(21) Application number: 10188748.7
(22) Date of filing: 25.10.2010
(51) Int. Cl.: A61K 9/00, A61K 31/192, A61P 31/00

(54) **Composition comprising anisic acid and an acid buffer**

(30) Priority: 23.10.2009 NL 1037411
(71) Applicant: PK Peters Krizman SA, 8001 Zürich (CH)
(72) Inventor: Peters, Albert Franjo, 5406 ND, UDEN (NL)
(74) Representative: Algemeen Octrooi- en Merkenbureau

(57) **Abstract**

The present invention relates to a composition, a form of dosage comprising this composition, and also the use of the composition or the form of dosage as a medicine.

It is an object of the present invention to provide a composition that prevents, reduces or cures infections, and promotes the growth of commensal microorganisms.

Another object of the present invention is to provide a composition that is suitable for use as an intimate product such as vaginal capsules.

## Description

The present invention relates to a composition, a form of dosage comprising said composition, and also the use of the composition or the form of dosage as a medicine.

A concentrated aqueous solution of anisic acid is disclosed in US 2006/0229291. Such a solution is used in cosmetic and dermatological formulations, the anisic acid being used to biologically stabilise the formulations. It is used in hair-care products to prevent the growth of microorganisms.

A microbicidal composition comprising anisic acid, a polyol and at least one cationic surfactant is disclosed in EP 1 325 731. Such a microbial composition is used in cosmetics, for example shampoo. These shampoo formulations do not contain an agent to promote adhesion.

These known documents describe the preservative effect of anisic acid and anisic acid combined with other compounds in cosmetic products.

Vaginal douches, also referred to as intimate products, are commercially available and comprise a solution that is applied internally to rinse the vagina. The aim of such douches is to prevent vaginal infections by making the vaginal environment more acidic through for example the presence of lactic acid in the solution.

These douches are not always easy to use, and are not always adequately effective either.

Also commercially available are tampons provided with a solution comprising lactic acid bacteria. During internal use these bacteria secrete lactic acid to influence the pH of the vagina, that is, to make it more acidic, to prevent vaginal infections. These tampons are however not always adequately effective.

The described vaginal douches and tampons both influence only the pH of the vagina, and so insufficiently prevent the growth of undesired microorganisms.

Also commercially available are vaginal moisturising gels, for example Gynofit. Gynofit moisturizing gel is an aqueous vaginal gel that forms a thin film of moisture on the vagina wall and moisturizes the mucous membrane. Gynofit comprises as a moisturizer glycerin. Gynofit further comprises hydroxyethylcellulose (HEC). HEC is used as a viscosity increaser and to gel the composition.

Vaginal capsules containing antifungal agents are known, for example dactarin. This agent controls fungal infections, such as vaginal infections due to Candida albicans. Miconazole damages the cell wall of the fungus, causing it to die. Another disadvantage of this agent is that it is only effective against fungal infections, and not against bacterial infections.

It is an object of the present invention to provide a composition that prevents, reduces or cures infections and promotes the growth of commensal microorganisms.

Another object of the present invention is to provide a composition that is suitable for use as an intimate product, for example in vaginal capsules.

One or more of the above mentioned objects are accomplished by a composition according to the introduction, which comprises anisic acid, a derivative thereof and/or a pharmaceutically acceptable salt hereof and an acid buffer.

The invention lies in the unique, novel combination of anisic acid and a buffer with a low pH. The term "buffer" as used herein is an acidity regulator, being an aqueous solution of at least two substances that are in a certain equilibrium and assume a certain pH. The pH will remain more or less constant on dilution of the solution in water, an acid or a base. This way, disturbance of the equilibrium and the acidity will be "buffered". An "acid buffer" consists of a weak acid and the salt of its conjugated base, and has a pH of less than 7. The buffer according to the present invention is for example present in an amount ranging between 1 and 50 wt.%.

The combination of anisic acid and acid buffer presents the advantage of preventing the growth of bacteria and fungi. Thanks to the presence of anisic acid that has an antifungal effect, the growth of fungi is extra effectively inhibited or even prevented. Thanks to the presence of an acid buffer the growth of both bacteria and fungi is inhibited.

Anisic acid (methoxybenzoic acid), a derivative thereof and/or a pharmaceutically acceptable salt hereof as present in the composition according to the present invention may for example be present in the form of an acid salt of an alkali, an alkali metal or an alkaline earth metal, in the form of an ammonium salt or a salt with an organic amine, or in the form of a C1-4 alkyl ester. The alkyl group of the ester may be linear or branched, and may be for example a methyl, ethyl, n-propyl, isopropyl, n-butyl or a tertiary butyl group. Examples of anisic-acid salts and esters are sodium anisate, potassium anisate, methyl anisate, ethyl anisate, propyl anisate or butyl anisate. Preferably, the composition according to the present invention contains para-anisic acid (4-methoxybenzoic acid). But meta-anisic acid (3-methoxybenzoic acid) may also be used.

Whenever mention is made of "anisic acid" in this text, this is also understood to include a derivative thereof and/or a pharmaceutically acceptable salt thereof, unless otherwise indicated.

In one preferred embodiment of the present invention the anisic acid, a derivative thereof and/or a pharmaceutically acceptable salt hereof is present in an amount ranging between 0.1 wt.% and 1.0 wt.%, preferably between 0.2 wt.% and 0.5 wt.%, in particular between 0.25 wt.% and 0.4 wt.%, relative to the total weight of the composition.

Anisic acid prevents or inhibits the growth of microorganisms such as fungi, for example yeasts, which are single-cell fungi. Compositions containing such an amount of anisic acid can be used on or in the body to prevent or inhibit the growth of microorganisms. The aforementioned amounts of anisic acid inhibit or prevent the growth of fungi (such as yeasts) without the anisic acid having a harmful or irritating effect on the body itself or on the part of the body to which the composition is applied. A person skilled in the art will be able to determine a suitable amount depending on the place and manner of application. Another advantage of anisic acid is that it also has an anti-inflammatory effect in the case of an existing infection. Anisic acid moreover masks any undesired odours that may be caused by an infection.

The composition of the present invention comprises an acid buffer. The buffer composition can be chosen so that the composition, if used as a medicine, will have a pH that will be optimum for a healthy microbiological flora. The desired pH will depend on the part of the body to which the composition is applied. The buffer preferably comprises an inorganic compound, in particular a salt of monohydrogen phosphate, for example Na2HP04.

In one embodiment according to the present invention the composition comprises a buffer with an organic acid, preferably citric acid, anisic acid or lactic acid, or a combination hereof, in particular at least citric acid as the acid component of the acid buffer.

In one embodiment of the present invention the composition comprises citric acid and a salt of monohydrogen phosphate. Such a multiple buffer composition is particularly advantageous because these ingredients have virtually no harmful effect on the body, and a good buffering effect is achieved.

A citrate-phosphate buffer can be set to the desired pH by adjusting the ratios of the monohydrogen phosphate and the citric acid. A large amount of citric acid with little monohydrogen phosphate is required for a low pH such as 2.5, whereas a large amount of monohydrogen phosphate combined with little citric acid must be present for a neutral pH. A person skilled in the art will be able to determine the right ratios, depending on the desired pH.

The buffer preferably has a pH lying in the pH range of the vagina.

Preferably the pH of the buffer ranges between 3 and 5.5. When the composition is applied on or in the body it will ensure a pH such that the growth of undesired microorganisms such as fungi (for example yeasts) and bacteria will be prevented. More preferably, the pH of the buffer ranges between 4 and 4.5. When the composition is applied in the vagina it will ensure a pH such that the growth of undesired microorganisms such as fungi (for example yeasts) and bacteria will be prevented.

In one embodiment according to the present invention a prebiotic is present, for example in an amount ranging between 1 and 80 wt.%. A prebiotic is an agent that selectively encourages the growth and/or activity of one or more types of bacteria, thus promoting good health. The prebiotic promotes the growth and maintenance of a healthy microbiological flora such as the commensal microorganisms that are present on and in the body, for example in the vagina. A healthy microbiological flora prevents the growth of infections with the risk of them becoming harmful for the body or the part of the body to which the composition is applied.

The prebiotic is preferably selected from the group comprising fructo-oligosaccharides(FOS), insulin, polydextrose, trans-galacto-oligosaccharides, xylo-oligosaccharides, manno-oligosaccharides, lactulose, lactitol or one or more combinations hereof, preferably lactitol. Lacitol is particularly preferable as it is very soluble.

In one embodiment of the present invention the composition comprises an agent to promote adhesion, for example in an amount ranging between 0.1 and 10 wt.%. An agent to promote adhesion is a bioadhesive compound or composition which adheres to biological tissue like the mucosa. This agent preferably is or comprises polyacrylic acid. Such an agent will ensure that when the composition is applied locally, for example in the vagina, it will not flow away from that area. This allows for the composition to deliver locally an active ingredient over a prolonged time. Polyacrylic acid exhibits very good adhesion to mucous membrane. This causes the composition according to the present invention to adhere well to the mucous membrane, for example in the vagina, to which it is applied, and it will then remain present in that area for a long time. The advantage of this is that the active ingredients in the composition will remain in the area where the activity of the ingredients is desired. This ensures an optimum therapeutic effect.

Polyacrylic acid namely adheres very well to mucous membrane. Because of this, the composition according to the present invention will adhere well to the mucous membrane for example in the vagina to which it is applied, and it will remain in this area for a long time.

The invention also relates to a form of dosage comprising the present composition. Bringing the composition into a form of dosage means that it can be used as a medicine. The fact that the composition can be applied in different forms of dosage means that it is possible to use the composition as a medicine for various disorders and in different parts of the body. The form of dosage is preferably selected from the group consisting of tablets, capsules, suppositories, gels, liquids for oral or intravenous use, ointments and creams.

The form of dosage is preferably a capsule. It is preferably a capsule that can be introduced into the vagina and will melt or dissolve in the vagina as a result of the local heat, causing the composition according to the present invention to be released to the surrounding area. This way the composition will be able to spread across the wall of the vagina, as a result of which the active ingredients will be distributed across the wall of the vagina. This will ensure an optimum local therapeutic effect.

The present invention also relates to use of the composition according to the present invention or of one or more of the forms of dosage according to the present invention as a medicine.

The present invention also relates to use of the composition according to the present invention or of one or more of the forms of dosage according to the present invention as a medicine against infection, preferably a vaginal infection, in particular a bacterial and/or fungal infection. The presence of anisic acid gives the composition a preventive and/or inhibiting effect on the growth of fungi, in particular yeasts. Very good results are achieved with anisic acid in the treatment of Candida infections, in particular when use is made of 4-methoxybenzoic acid.

If the composition according to the present invention is used in a capsule as a medicine against infections, in particular vaginal infections as described above, then it is preferable for the capsule to be introduced into a patient's vagina before she goes to sleep at night. As the patient will then be prostate, a minimal amount of the composition will flow away and be lost - something that is promoted by a patient's upright posture. This will ensure an optimum local therapeutic effect.

In one embodiment the invention furthermore relates to a composition comprising anisic acid, a derivative thereof and/or a pharmaceutically acceptable salt hereof and a prebiotic. The prebiotic is preferably selected from the group consisting of fructo-oligosaccharides (FOS), inulin, polydextrose, trans-galacto-oligosaccharides, xylo-oligosaccharides, manno-oligosaccharides, lactulose, lactitol or one or more combinations hereof, preferably lactitol. In this embodiment the invention furthermore relates to a composition that furthermore comprises an agent to promote adhesion, for example polyacrylic acid. In this embodiment the invention moreover relates to a composition that comprises an acid buffer that preferably comprises an inorganic compound, in particular a monohydrogen phosphate. The buffer in this embodiment furthermore preferably comprises an organic acid, preferably citric acid, acetic acid or lactic acid, or a combination hereof, in particular at least citric acid. The buffer may also comprise an inorganic compound and an organic acid. This composition can be used in the form of dosage described above to prevent the disorders described above.

### Example 1

| mass % | Ingredient |
|---|---|
| 0,30 | Dermosoft 688 ECO; p-anisic acid |
| 2,00 | Magnesiumstearate |
| 3,00 | Polycarbophil = Noveon AA-1 |
| 10,00 | Na-CMC = Blanose 9H4XF Pharm |
| 65,25 | Lactitolmonohydrate crystalline |
| 10,39 | Acidum citricum anhydricum |
| 9,06 | Di-potasium-1H-fosfate 17,8% P 44,8% K |

The present inventors have found that the composition according to table 1 has good adhesion when applied to the vagina and adheres better that compositions according to the state of the art. Further the inventors have found that a composition according to table 1 has improved activity against fungal infection compared to compositions according to the state of the art.

## Claims

1. A composition comprising anisic acid, a derivative thereof and/or a pharmaceutically acceptable salt hereof and an acid buffer.

2. A composition according to the preceding claim, **characterised in that** the composition comprises a prebiotic.

3. A composition according to either one of the preceding claims, **characterised in that** the prebiotic is selected from the group consisting of fructo-oligosaccharids (FOS), inulin, polydextrose, trans-galacto-oligosaccharides, xylo-oligosaccharides, manno-oligosaccharides, lactulose, lactitol or one or more combinations hereof, preferably lactitol.

4. A composition according to any one of the preceding claims, **characterised in that** it furthermore comprises an agent to promote adhesion, said agent preferably comprising polyacrylic acid.

5. A composition according to any one of the preceding claims, **characterised in that** the acid buffer comprises an inorganic compound.

6. A composition according to claim 5, **characterised in that** the inorganic compound is a monohydrogen phosphate.

7. A composition according to any one of the preceding claims, **characterised in that** the acid buffer comprises an organic acid, preferably citric acid, acetic acid or lactic acid, or a combination hereof, in particular at least citric acid.

8. A composition according to claim 7, **characterised in that** the buffer comprises an inorganic compound and an organic acid, preferably monohydrogen phosphate and citric acid.

9. A composition according to any one of the preceding claims, **characterised in that** the composition comprises between 0.1 wt.% and 1.0 wt.%, preferably between 0.2 wt.% and 0.5 wt.%, in particular between 0.25 wt.% and 0.4 wt.% anisic acid, a derivative thereof and/or a pharmaceutically acceptable salt thereof, relative to the total weight of the composition.

10. Form of dosage comprising the composition according to one or more of the preceding claims.

11. Form of dosage according to claim 10, selected from the group consisting of capsules, tablets or gels, preferably a capsule.

12. A composition according to one or more of claims 1-9 as a medicine.

13. Use of the composition according to one or more of claims 1-9 or of the form of dosage according to either one or both of claims 10 and 11 as a medicament against an infection, preferably a vaginal infection, in particular a bacterial and/or fungal infection.

14. Use according to claim 13, **characterised in that** the fungal infection is a yeast infection, preferably a Candida infection.
